(19) Europäisches Patentamt
European Patent Office
Office européen des brevets

(11) Veröffentlichungsnummer: **0 192 034 B1**

(12) **EUROPÄISCHE PATENTSCHRIFT**

(45) Veröffentlichungstag der Patentschrift: **15.07.92**

(51) Int. Cl.[5]: **C07D 223/18**, A01N 43/46

(21) Anmeldenummer: **86100288.9**

(22) Anmeldetag: **10.01.86**

Die Akte enthält technische Angaben, die nach dem Eingang der Anmeldung eingereicht wurden und die nicht in dieser Patentschrift enthalten sind.

# (54) Dibenzazepinderivate, deren Herstellung, zu deren Herstellung dienende Ausgangsmaterialien und Verwendung der Dibenzazepinderivate als Schädlingsbekämpfungsmittel.

(30) Priorität: **10.01.85 CH 95/85**
**28.10.85 CH 4629/85**

(43) Veröffentlichungstag der Anmeldung:
**27.08.86 Patentblatt 86/35**

(45) Bekanntmachung des Hinweises auf die Patenterteilung:
**15.07.92 Patentblatt 92/29**

(84) Benannte Vertragsstaaten:
**AT BE CH DE FR GB IT LI NL SE**

(56) Entgegenhaltungen:
**CH-A- 367 829**

(73) Patentinhaber: **CIBA-GEIGY AG**
**CH-4002 Basel(CH)**

(72) Erfinder: **Bruderer, Hans, Dr.**
**Schulgasse 4**
**CH-4105 Biel-Benken(CH)**
Erfinder: **Zurflüh, René, Dr.**
**Dachslenbergstrasse 54**
**CH-8180 Bülach(CH)**

**EP 0 192 034 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus CH-A-367829 sind Dibenzazepinederivate beschrieben, die z.B. als Vasodilatoren verwenden werden können.

Die vorliegende Erfindung betrifft heterocyclische Verbindungen, und zwar 5,7-Dihydro-6H-dibenz[c,e]-azepin-6-(thio)carboximidsäureester der allgemeinen Formel

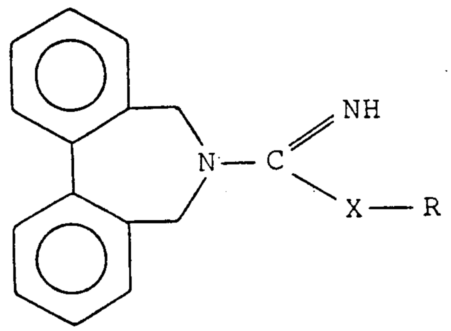

I

worin

R $C_{1-12}$-Alkyl, $C_{3-12}$-Alkenyl, $C_{3-12}$-Alkinyl oder $C_{3-8}$-Cycloalkyl und

X Sauerstoff oder Schwefel bedeuten,

und keine der allfälligen Mehrfachbindungen von R in $\alpha$-Stellung zu X steht, sowie Säureadditionssalze dieser Verbindungen.

Die erfindungsgemässen Verbindungen, d.h. die Verbindungen der Formel I und ihre Säureadditionssalze, sind Schädlingsbekämpfungsmittel und eignen sich insbesondere zur Bekämpfung von Insekten und Milben, z.B. Spinnmilben. Somit umfasst die Erfindung auch Schädlingsbekämpfungsmittel, welche erfindungsgemässe Verbindungen als Wirkstoffe enthalten, ein Verfahren zur Herstellung dieser Verbindungen sowie die Verwendung dieser Verbindungen bzw. Mittel zur Bekämpfung von Schädlingen.

Die in obiger Definition der Verbindungen der Formel I erwähnten $C_{1-12}$-Alkyl-, $C_{3-12}$-Alkenyl-und $C_{3-12}$-Alkinylreste können sowohl geradkettig als auch verzweigt sein. Zudem können die $C_{3-12}$-Alkenyl-und $C_{3-12}$-Alkinylreste eine oder mehrere Doppel- bzw. Dreifachbindungen aufweisen.

Falls in den Verbindungen der Formel I asymmetrische Kohlenstoffatome vorliegen, treten die Verbindungen in optisch aktiver Form auf. Im Falle derjenigen Verbindungen der Formel I, in denen R $C_{3-12}$-Alkenyl bedeutet, kann zusätzlich geometrische Isomerie auftreten. Die Formel I soll demnach all diese möglichen isomeren Formen sowie deren Gemische, z.B. racemische Gemische, umfassen.

Als Säureadditionssalze der Verbindungen der Formel I kommen physiologisch verträgliche Salze in Frage. Hierzu gehören Salze dieser Verbindungen mit anorganischen und organischen Säuren, vorzugsweise Halogenwasserstoffsäuren, wie Salzsäure und Bromwasserstoffsäure; Salpetersäure; Phosphorsäure; Schwefelsäure; mono- und bifunktionellen Carbonsäuren und Hydroxycarbonsäuren, wie Essigsäure, Maleinsäure, Bernsteinsäure, Fumarsäure, Weinsäure, Zitronensäure, Salicylsäure, Sorbinsäure und Milchsäure; und Sulfonsäuren, wie 1,5-Naphthalin-disulfonsäure.

Unabhängig voneinander bedeuten R vorzugsweise $C_{1-12}$-Alkyl, insbesondere $C_{1-3}$-Alkyl, und X vorzugsweise Sauerstoff. Besonders bevorzugte Verbindungen der Formel I sind:

5,7-Dihydro-6H-dibenz[c,e]azepin-6-carboximidsäureäthylester und dessen Hydrochlorid,

5,7-Dihydro-6H-dibenz[c,e]azepin-6-carboximidsäuremethylester und dessen Hydrochlorid,

5,7-Dihydro-6H-dibenz[c,e]azepin-6-carboximidsäurepropylester und dessen Hydrochlorid und

5,7-Dihydro-6H-dibenz[c,e]azepin-6-carboximidsäureisopropylesterund dessen Hydrochlorid.

Das erfindungsgemässe Verfahren zur Herstellung der Verbindungen der Formel I und von ihren Säureadditionssalzen ist dadurch gekennzeichnet, dass man

a) 5,7-Dihydro-6H-dibenz[c,e]azepin-6-carbonitril, d.h. das Cyanamid der Formel

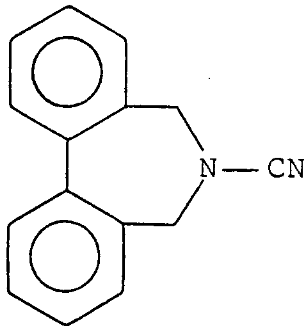

II

mit einem Alkohol bzw. Thiol der allgemeinen Formel

R-XH III

worin R und X die oben angegebenen Bedeutungen besitzen, oder mit einem Alkalimetallsalz davon umsetzt, oder
b) zwecks Herstellung derjenigen Verbindungen der Formel I, in denen R Methyl oder Aethyl bedeutet, einen Harnstoff bzw. Thioharnstoff der allgemeinen Formel

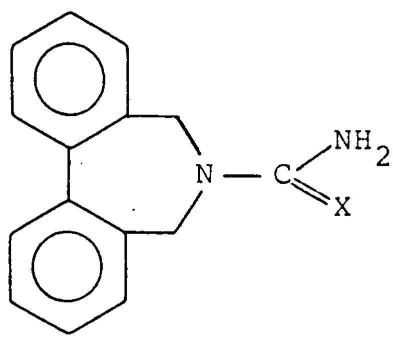

IV

worin X die oben angegebene Bedeutung besitzt, alkyliert,
und erwünschtenfalls eine erhaltene Verbindung der Formel I durch Umsetzung mit einer Säure in das entsprechende Säureadditionssalz überführt.

Die Umsetzung nach Verfahrensvariante a) erfolgt zweckmässigerweise unter Verwendung überschüssigen Alkohols bzw. Thiols der Formel III als Lösungsmittel und in Gegenwart einer katalytischen oder stöchiometrischen Menge eines Alkalimetallsalzes, insbesondere des Natrium- oder Kaliumsalzes, des Alkohols oder Thiols der Formel III . In einer weiteren Ausführungsform werden der Alkohol bzw. das Thiol der Formel II als Reaktionspartner und ein Aequivalent Alkalimetallcyanid, insbesondere Natrium- oder Kaliumcyanid, verwendet. Die Reaktionstemperaturen können in einem grossen Bereich variiert werden, und zwar im allgemeinen von 10 bis 80 ° C, vorzugsweise von 20 bis 60 ° C.

Die Alkylierung nach Verfahrensvariante b) wird zweckmässigerweise unter Verwendung von Trimethyl- bzw. Triäthyl-oxoniumtetrafluoroborat, vorzugsweise mittels mindestens eines Aequivalents dieser Mittel und vorzugsweise in einem chlorierten Kohlenwasserstoff, wie Methylenchlorid oder Chloroform, als Lösungsmittel durchgeführt. Die Reaktionstemperaturen liegen zweckmässigerweise zwischen 0 und 40 ° C, vorzugsweise zwischen 20 und 25 ° C. Das so gebildete Salz der Verbindung der Formel I kann durch Behandlung mit einer Base, vorzugsweise Natriumcarbonat in wässriger Lösung, auf konventionelle Weise in den freien Thiocarboximidsäureester übergeführt werden.

Sofern keine gezielte Synthese zur Isolierung reiner Isomerer durchgeführt wird, fällt das Produkt im Falle des Vorhandenseins zweier oder mehrerer Isomerer als Gemisch dieser Isomeren an. Die Isomeren können nach an sich bekannten Methoden aufgetrennt werden, oder sie können gewünschtenfalls auch z.B. durch Synthese aus entsprechenden optisch aktiven Ausgangsmaterialien hergestellt werden.

Zur Herstellung der Säureadditionssalze der Verbindungen der Formel I werden die Verbindungen I mit den gewünschten Säuren auf übliche Weise umgesetzt, beispielsweise durch Lösen der Verbindung der

Formel I in einem geeigneten Lösungsmittel und Hinzufügen der Säure.

Die Isolierung und die Reinigung der so hergestellten Verbindungen der Formel I bzw. der Säureadditionssalze können nach an sich bekannten Methoden erfolgen.

Das als Ausgangsmaterial in der Verfahrensvariante a) verwendete Cyanamid der Formel II ist neu und bildet einen weiteren Gegenstand der vorliegenden Erfindung. Dieses Ausgangsmaterial kann beispielsweise hergestellt werden, indem man

a') eine Verbindung der allgemeinen Formel

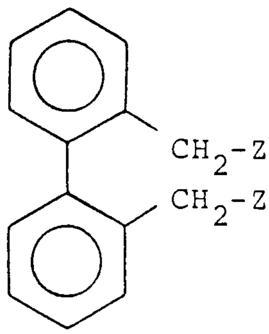

V

worin Z eine Abgangsgruppe, wie Halogen (vorzugsweise Chlor oder Brom), Mesyloxy oder Tosyloxy, bedeutet,

mit dem Dinatrium- oder Calciumsalz von Cyanamid in einem Alkohol, z.B. Aethanol, oder mit Cyanamid selbst in Gegenwart von Dimethylsulfoxid-Natrium in Dimethylsulfoxid, oder mit Cyanamid in Gegenwart von 50%-iger Natronlauge, einem inerten, mit Wasser nichtmischbaren Lösungsmittel, wie einem Aromaten, z.B. Benzol oder Toluol, einem aliphatischen oder cyclischen Aether, z.B. tert.Butyl-methyläther, oder einem halogenierten Kohlenwasserstoff, z.B. Methylenchlorid, und einem Phasentransferkatalysator, wie Tricaprylmethylammoniumchlorid oder Tetrabutylammoniumchlorid, umsetzt.

Bei dieser Verfahrensvariante können die Reaktionstemperaturen in einem grossen Bereich, wie zwischen 20 ° C und der Rückflusstemperatur des Reaktionsgemisches, variiert werden. Die Verbindungen der Formel V sind ihrerseits entweder bekannt oder können nach an sich bekannten Methoden aus 2,2'-Biphenylmethanol hergestellt werden.

Weitere Verfahren zur Herstellung des Ausgangsmaterials der Formel II bestehen darin, dass man

b') 6,7-Dihydro-5H-dibenz[c,e]azepin mit einem Halogencyan, vorzugsweise Chlor- oder Bromcyan, bei tiefen Temperaturen, vorzugsweise -5 bis 10 ° C, in einem inerten Lösungsmittel, wie einem aliphatischen oder cyclischen Aether, einem Aromaten oder einem halogenierten Kohlenwasserstoff, z.B. Chloroform, umsetzt, oder

c') einen Harnstoff bzw. Thioharnstoff der Formel IV mit einer überschüssigen Menge Chloroform und 50%-iger Natronlauge in Gegenwart eines Phasentransferkatalysators, vorzugsweise eines tertiären Amins, wie Triäthylamin, bei Raumtemperatur und unter Rühren behandelt, wobei Wasser bzw. Schwefelwasserstoff aus dem N-Substituenten von IV eliminiert wird.

Noch weitere Verfahren zur Herstellung des Ausgangsmaterials der Formel II basieren auf den in Houben-Weyl, Methoden der Organischen Chemie, Bd. VIII, Seiten 172-177, beschriebenen allgemeinen Methoden.

Auch die Ausgangsmaterialien der Formel IV sind neu und bilden einen weiteren Gegenstand der vorliegenden Erfindung. Sie können beispielsweise durch Erhitzen von 6,7-Dihydro-5H-dibenz[c,e]azepin mit Harnstoff bzw. Thioharnstoff auf 130-150 ° C oder durch Umsetzung des Azepin-Hydrochlorids mit Kaliumcyanat bzw. -thiocyanat bei leicht erhöhter Temperatur, vorzugsweise bei 50-80 ° C, hergestellt werden. Diese Methoden sind an sich bekannt, z.B. aus Houben-Weyl, Methoden der Organischen Chemie, Bd. VIII, Seiten 149-157. Ein besonders bevorzugtes Ausgangsmaterial der Formel IV ist das 5,7-Dihydro-6H-dibenz[c,e]-azepin-6-carboxamid.

Die Isolierung und die Reinigung der so hergestellten Ausgangsmaterialien können nach an sich bekannten Methoden durchgeführt werden.

Die Verbindungen der Formel I sind ganz allgemein als Pestizide von Wert. Sie erweisen sich als besonders wertvoll zur Bekämpfung von Milben und Insekten, insbesondere von

- Milben, die bei dem Pflanzenschutz von Bedeutung sind, wie z.B.
  Tetranychidae (Spinnmilben), insbesondere Tetranychus urticae, Tetranychus cinnabarinus, Tetranychus turkestani, Tertranychus McDanieli, Tetranychus kanzawai;

Panonychus ulmi, Panonychus citri;
Phyllocoptruta oleivora;
Aculus schlechtendali;
Phyllocoptes vitis;
Aceria essigi, Aceria gracilis;
Cecidophyopsis ribis;
Eriophyes vitis, Eriophyes sheldoni, Eriophyes tulipae;
Eotetranychus sexmaculatus, Eotetranychus carpini;
Hemitarsonemus latus;
Acarus siro;
Bryobia graminum;

- Milben, die in der Veterinärmedizin von Bedeutung sind, wie z.B.
  Macronyssus bursa, Macronyssus sylviarum, Macronyssus lacoti;
  Dermanyssus gallinae;
  Zecken, insbesondere der Familien Ixodidae und Argasidae und der Ordnungen Boophilus, Amblyomma, Hyalomma,
  Rhipicephalus, Ixodes, Argas und Ornithodorus.

Die erfindungsgemässen Verbindungen wirken als Kontakt-und Frassgifte. Zudem werden einige der Verbindungen von verschiedenen Pflanzen aufgenommen, so dass die zu bekämpfenden Schädlinge beim Fressen der Pflanzen vernichtet werden. Diese Verbindungen weisen also systemische Wirkung auf.

Das erfindungsgemässe Schädlingsbekämpfungsmittel ist dadurch gekennzeichnet, dass es eine wirksame Menge mindestens einer Verbindung der allgemeinen Formel I, wie oben definiert, oder eines Säureadditionssalzes einer solchen Verbindung sowie Formulierungshilfsstoffe enthält. Das Mittel enthält zweckmässigerweise zumindest einen der folgenden Formulierungshilfsstoffe:

Feste Trägerstoffe; Lösungs- bzw. Dispersionsmittel; Tenside (Netz- und Emulgiermittel); Dispergatoren (ohne Tensidwirkung); und Stabilisatoren.

Unter Verwendung dieser und gegebenenfalls zusätzlicher Hilfsstoffe können die Verbindungen der Formel I, also die pestiziden Wirkstoffe, in die üblichen Formulierungen übergeführt werden, wie Lösungen, Suspensionen, Emulsionen, emulgierbare Konzentrate, Pasten, Schäume, Stäube, Pulver und Granulate.

Als feste Trägerstoffe kommen im wesentlichen in Frage: natürliche Mineralstoffe, wie Kaolin, Tonerden, Kieselgur, Talkum, Bentonit, Kreide, Kalkstein, Quarz, Dolomit, Attapulgit, Montmorillonit und Diatomeenerde; synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Stäube, Pulver oder Granulate vorliegen können.

Als Lösungs- bzw. Dispersionsmittel kommen im wesentlichen in Frage: Aromaten, wie Toluol, Xylole, Benzol und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungsmittel, wie Dimethylformamid, N-Methylpyrrolidon und Dimethylsulfoxid, wobei solche Lösungs- bzw. Dispersionsmittel vorzugsweise Flammpunkte von mindestens 30 °C und Siedepunkte von mindestens 50 °C aufweisen, und Wasser. Als Lösungs- bzw. Dispersionsmittel kommen auch sogenannte verflüssigte gasförmige Streckmittel oder Trägerstoffe in Frage, also Produkte, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Im Falle der Benutzung von Wasser als Lösungsmittel können z.B. auch organische Lösungsmittel als Hilfslösungsmittel verwendet werden.

Die Tenside (Netz- und Emulgiermittel) können nicht-ionische Verbindungen darstellen, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-Dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen darstellen, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäure, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eigen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butylhydroxytoluol; UV-Absorber, z.B. substituierte Benzophenone, Diphenylacrylnitrilsäureester und Zimtsäureester; und Deaktivatoren, z.B. Salze der Aethylendiaminotetraessigsäure und Polyglykole.

Die erfindungsgemässen Schädlingsbekämpfungsmittel können neben den Wirkstoffen der Formel I auch andere Wirkstoffe enthalten, z.B. anderweitige Schädlingsbekämpfungsmittel, Schädlingslockstoffe, Fungizide, Bakterizide, Pflanzenwachstumsregulatoren und Düngemittel. Solche Kombinationsmittel eignen sich zur Verstärkung der Aktivität bzw. zur Verbreiterung des Wirkungsspektrums. Gegebenenfalls können dadurch auch Unzulänglichkeiten von bisher bekannten zugesetzten Mitteln ausgeglichen werden.

Es wurde gefunden, dass die erfindungsgemässen Verbindungen I, insbesondere die besonders bevorzugte Verbindung 5,7-Dihydro-6H-dibenz[c,e,]azepin-6-carboximidsäure-äthylester und dessen Hydrochlorid, mit Vorteil in Kombination mit konventionellen Akariziden, insbesondere mit zur Bekämpfung von Eiern und Larven von Milben geeigneten konventionellen Akariziden, eingesetzt werden. Beispiele derartiger Akarizide sind Chlorbenside, Chlorfenson, Clofentezine, Fenson, Fenothiocarb, Flubenzimine, Tetradifon, Hexythiazox, Benzoximate, Amitraz, Dienochlor und 4-Pent-4-ynyloxyphenyl-phenyl-äther sowie 1,2,4-Triazole mit akarizider Wirkung, wie beispielsweise 3-(o-Chlorphenyl)-1-methyl-5-(o-trifluormethyl-phenyl)-1H -1,2,4-triazol. Die Anwendung kann gleichzeitig oder getrennt erfolgen. Dabei können die erfindungsgemässen Wirkstoffe den Nachteil bekannter Akarizide mit Wirkungsschwerpunkt gegen die Eier und Larven kompensieren, indem die nach Einsatz dieser bekannten Akarizide überlebenden mobilen Stadien, welche sich rasch zu einer neuen schädlichen Population entwickeln können, ebenfalls abgetötet werden. Da unter Praxisbedingungen oft Eier, verschiedene Larvendstadien sowie Adulte, und zwar männliche und weibliche Formen, gleichzeitig auftreten, kann mit Kombinationspräparaten äusserst praxisbezogene, d.h. raschere, durchschlagendere und länger anhaltende Gesamtwirkung erzielt werden. Die totale Menge der beiden Wirkstoffe in solchen Kombinationspräparaten beträgt aber zweckmässigerweise nicht mehr als die Menge Wirkstoff bei Verwendung einer Verbindung I als alleinigen Wirkstoff.

Die erfindungsgemässen Schädlingsbekämpfungsmittel enthalten je nach Typ zwischen 0,005 und 95 Gewichtsprozent erfindungsgemässer Verbindung bzw. erfindungsgemässer Verbindungen als Wirkstoff. Sie können in einer Form vorliegen, die sich für die Lagerung und den Transport eignet. In solchen Formen, z.B. emulgierbaren Konzentraten, ist die Wirkstoffkonzentration normalerweise im höheren Bereich der obigen Konzentrationsreihe. Diese Formen können dann mit gleichen oder verschiedenen Formulierungshilfsstoffen bis zu Wirkstoffkonzentrationen verdünnt werden, die sich für den praktischen Gebrauch eignen, und solche Konzentrationen liegen normalerweise im niedrigeren Bereich der obigen Konzentrationsreihe. Emulgierbare Konzentrate enthalten in allgemeinen 5 bis 90 Gewichtsprozent, vorzugsweise 10 bis 80 Gewichtsprozent, der erfindungsgemässen Verbindung(en). Als Anwendungsformen kommen u.a. gebrauchsfertige Lösungen, Emulsionen, Suspensionen, Schäume, Pulver, Pasten, Stäubemittel und Granulate in Frage. Die Wirkstoffkonzentrationen in solchen anwendungsfertigen Zubereitungen können in grossen Bereichen variiert werden. In Sprizbrühen können z.B. Konzentrationen zwischen 0,005 und 0,5 Gewichtsprozent vorliegen. Im Ultra-Low-Volume-Verfahren können Spritzbrühen formuliert werden, in denen die Wirkstoffkonzentration vorzugsweise von 10 bis 20 Gewichsprozent beträgt, während die im Low-Volume-Verfahren und im High-Volume-Verfahren formulierten Spritzbrühen vorzugsweise eine Wirkstoffkonzentration von 0,01 bis 0,5 bzw. 0,005 bis 0,1 Gewichtsprozent aufweisen. Granulate enthalten vorzugsweise von 5 bis 50 Gewichtsprozent der erfindungsgemässen Verbindung(en) als Wirkstoff.

Die erfindungsgemässen Schädlingsbekämpfungsmittel können dadurch hergestellt werden, dass man mindestens eine Verbindung der allgemeinen Formel I bzw. ein Säureadditionssalz einer solchen Verbindung mit Formulierungshilfsstoffen vermischt.

Die Herstellung der Mittel kann in bekannter Weise durchgeführt werden, z.B. durch Vermischen des Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln oder von Dispergatoren, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem festen Trägerstoff vermischt werden, z.B. durch Zusammenmahlen; oder man kann den festen Trägerstoff mit einer Lösung oder Suspension des Wirkstoffes imprägnieren und dann das Lösungs- oder Suspensionsmittel durch Abdunsten, Erhitzen oder durch Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die Verbindungen der Formel I bzw. deren Säureadditionssalze können auch mit einem Tensid und einem festen Trägerstoff zur Bildung eines netzbaren Pulvers vermischt werden, welches in Wasser dispergierbar ist, oder sie können mit einem festen granulierten Trägerstoff zur Bildung eines Granulates vermischt werden.

Wenn gewünscht, kann die Verbindung der Formel I oder ein Säureadditionssalze davon in einem mit Wasser nicht mischbaren Lösungsmittel, wie beispielsweise einem alicyclischen Keton, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Das erfindungsgemässe Verfahren zur Bekämpfung von Schädlingen ist dadurch gekennzeichnet, dass man das zu schützende Gut oder die Schädlinge selbst mit einer wirksamen Menge einer erfindungsgemässen Verbindung bzw. eines erfindungsgemässen Schädlingsbekämpfungsmittels behandelt. Dieses Anwendungsverfahren kann durch Boden- oder Blattapplikation, bzw. durch Applikation auf die zu schützenden Tiere, Vorräte oder Materialien, je nach Art der zu bekämpfenden Schädlinge, durchgeführt werden. Die Bekämpfung wird beispielsweise durch Kontakt oder durch Einnahme mit der Nahrung erzielt.

Die Anwendung kann in konventioneller Weise geschehen, z.B. durch Verspritzen, Versprühen, Vernebeln, Verstäuben, Verstreuen, Eindrillen, Verräuchern, Giessen, Beizen oder Inkrustieren. Pulverförmige Präparate können z.B. als Stäubemittel mit Hilfe der üblichen Verstäubegeräte auf die Schädlinge bzw. auf das zu schützende Gut, z.B. Pflanzen oder Tiere, aufgebracht werden. Wässrige Suspensionen sind z.B. als Spritzmittel anwendbar.

Bei der Anwendung im Pflanzenschutz genügt in der Regel eine Dosierung von ca. 100-500 g Wirkstoff [Verbindungen(en) der Formel I]/ha, z.B. wie dies bei der Applikation von 2000 1 einer Spritzbrühe, die 0,005-0,025 Gewichtsprozent Wirkstoff enthält, auf 1 ha bepflanzten Erdbodens der Fall ist.

Die nachfolgenden Beispiele dienen zur näheren Erläuterung der Erfindung.

## I. Herstellung der Wirkstoffe der Formel I:

### Beispiel 1

Zu einer aus 0,16 g Natrium und 20 ml absolutem Methanol hergestellten Lösung von Natriummethylat in Methanol werden 1,5 g 5,7-Dihydro-6H-dibenz[c,e]azepin-6-carbonitril gegeben, und das Gemisch wird während 1 Stunde auf Rückflusstemperatur erhitzt. Anschliessend wird das Lösungsmittel abdestilliert, der Rückstand mit Wasser versetzt und das resultierende wässrige Gemisch dreimal mit Methylenchlorid extrahiert. Man wäscht die vereinigten Extrakte mit Wasser, trocknet sie über wasserfreiem Natriumsulfat und dampft sie ein. Auf diese Weise erhält man den 5,7-Dihydro-6H-dibenz[c,e]azepin -6-carboximidsäure-methylester als Oel.

Man löst das obige Produkt in wenig Alkohol und versetzt die Lösung mit 1,4 ml einer 5N alkoholischen Salzsäure-Lösung. Dann gibt man 20 ml n-Hexan zu, nutscht das ausgefallene Produkt ab und kristallisiert es aus Alkohol um. Man erhält reines 5,7-Dihydro-6H-dibenz[c,e]azepin -6-carboximidsäure-methylester-hydrochlorid, Smp. 205-208 °C.

In analoger Weise erhält man ausgehend von

- 5,7-Dihydro-6H-dibenz[c,e]azepin-6-carbonitril und Natriumäthylat in Aethanol den 5,7-Dihydro-6H-dibenz[c,e]azepin -6-carboximidsäure-äthylester und daraus mit Salzsäure in Alkohol das Hydrochlorid, Smp. 158 °C (unter Zersetzung).
- 5,7-Dihydro-6H-dibenz[c,e]azepin-6-carbonitril und Natriumäthanthiolat in Aethanthiol den 5,7-Dihydro-6H-dibenz[c,e]azepin -6-thiocarboximidsäure-äthylester und daraus mit Salzsäure in Alkohol das Hydrochlorid, Smp. 220 °C (unter Zersetzung).
- 5,7-Dihydro-6H-dibenz[c,e]azepin-6-carbonitril und Natriumpropylat in n-Propanol den 5,7-Dihydro-6H-dibenz[c,e]azepin-6-carboximidsäure-propylester und daraus mit Salzsäure in Alkohol das Hydrochlorid, Smp. 147 ° C (unter Zersetzung).

- 5,7-Dihydro-6H-dibenz[c,e]azepin-6-carbonitril und Natriumisopropylat in Isopropanol den 5,7-Dihydro-6H-dibenz[c,e]azepin -6-carboximidsäure-isopropylester und daraus mit Salzsäure in Alkohol das Hydrochlorid, Smp. 158 ° C (unter Zersetzung).
- 5,7-Dihydro-6H-dibenz(c,e)azepin-6-carbonitril und Natriumdodecanthiolat in 1-Dodecanthiol den 5,7-Dihydro-6H-dibenz[c,e]azepin -6-thiocarboximidsäure-dodecylester, der durch Flash-Chromatographie an Kieselgel unter Verwendung von n-Hexan/Aethylacetat (1:1) als Laufmittel gereinigt wird; $^1$H-NMR-($CDCl_3$): 0,88 (t, $CH_3$), 1,0-2,0 (2OH), 2,5 (t, $SCH_2$), 4,43 (s, 4H), 7,3-7,7 (8H).
- 5,7-Dihydro-6H-dibenz[c,e]azepin-6-carbonitril und Natrium-2-butanthiolat in 2-Butanthiol den 5,7-Dihydro-6H-dibenz[c,e]azepin -6-thiocarboximidsäure-2-butylester, der durch Flash-Chromatographie an Kieselgel unter Verwendung von n-Hexan/Aethylacetat (1:1) als Laufmittel gereinigt wird, $^1$H-NMR-($CDCl_3$): 1,05 (t,$CH_2CH_3$), 1,42 (d, CH-$CH_3$), 1,46-1,93 (m, $CH_2CH_3$), 2,81-3,37 (m, 1H), 4,41 (s, 4H), 5,1 (breites s, NH), 7,37-7,70 (8H).

Beispiel 2

Ein Gemisch von 1,99 g 5,7-Dihydro-6H-dibenz[c,e]azepin-6-carbonitril, 0,59 g Kaliumcyanid und 20 ml Allylalkohol wird während 20 Stunden bei Raumtemperatur gerührt und danach das Allylalkohol abdestilliert. Der Rückstand wird auf Eiswasser gegossen und mit Methylenchlorid extrahiert, und die vereinigten Extrakte werden mit Wasser gewaschen, über wasserfreiem Natriumsulfat getrocknet und eingedampft. Nach Flash-Chromatographie an Kieselgel (Laufmittel: Aethylacetat) erhält man reinen 5,7-Dihydro-6H-dibenz[c,e]azepin-6-carboximidsäure-allylester als Oel.

Man löst das obige Produkt in 2,5 ml Methanol, kühlt die Lösung auf 0 ° C und versetzt sie mit 1,3 ml einer 5N alkoholischen Salzsäure-Lösung. Nach 15-minütigem Rühren werden 10 ml n-Hexan zugegeben, und das ausgefallene Produkt wird dann abgenutscht. Man erhält 5,7-Dihydro-6H-dibenz[(c,e]azepin -6-carboximidsäure-allylester-hydrochlorid, Smp. 115 ° C (unter Zersetzung).

In analoger Weise erhält man ausgehend von

- 5,7-Dihydro-6H-dibenz[c,e]azepin-6-carbonitril und n-Butanol den 5,7-Dihydro-6H-dibenz[c,e]azepin -6-carboximidsäure-butylester als harzartiges Produkt, $^1$H-NMR($CDCl_3$): 1,02 (t, $CH_2CH_3$), 1,4-2,0 (4H), 4,15 (t, $OCH_2$), 4,27 (s, 4H), 4,57 (breites s, NH), 7,3-7,7 (8H).
- 5,7-Dihydro-6H-dibenz[c,e]azepin-6-carbonitril und 2-Butanol den 5,7-Dihydro-6H-dibenz[c,e]azepin -6-carboximidsäure-2-butylester als harzartiges Produkt, $^1$H-NMR($CDCl_3$): 1,02 (t, $CH_2CH_3$), 1,35 (d, CH-$CH_3$), 1,4-2,0 (m, 2H), 4,23 (s, 4H), 4,83 (m, 1H), 7,3-7,7 (8H).
- 5,7-Dihydro-6H-dibenz[c,e]azepin-6-carbonitril und Isobutanol den 5,7-Dihydro-6H-dibenz[c,e]azepin -6-carboximidsäure-isobutylester als harzartiges Produkt, $^1$H-NMR($CDCl_3$): 1,07 [d, $CH(CH_3)_2$], 2,1 (m, 1H), 3,95 (d, $CH_2$), 4,25 (s, 4H), 4,55 (breites s, NH), 7,3-7,7 (8H).
- 5,7-Dihydro-6H-dibenz[c,e]azepin-6-carbonitril und 1-Octanol den 5,7-Dihydro-6H-dibenz[c,e]azepin -6-carboximidsäure-octylester als harzartiges Produkt, $^1$H-NMR($CDCl_3$): 0,88 (t, $CH_3$), 1,0-2,0 (12H), 4,15 (t, $OCH_2$), 4,23 (s, 4H), 4,87 (breites s, NH), 7,3-7,7 (8H).
- 5,7-Dihydro-6H-dibenz[c,e]azepin-6-carbonitril und Propargylalkohol den 5,7-Dihydro-6H-dibenz-[c,e] -6-carboximidsäure-2-propinylester als harzartiges Produkt, $^1$H-NMR($CDCl_3$): 2,51 (t, CH), 4,2 (s, 4H), 4,41 (breites s, NH), 4,87 (d, $OCH_2$), 7,3-7,7 (8H).
- 5,7-Dihydro-6H-dibenz[c,e]azepin-6-carbonitril und Cyclohexanol den 5,7-Dihydro-6H-dibenz[c,e]-azepin -6-carboximidsäure-cyclohexylester als harzartiges Produkt, $^1$H-NMR($CDCl_3$): 1,1-2,3 (1OH), 4,21 (s, 4H), 4,75 (m, OCH), 4,95 (breites s, NH), 7,3-7,7 (8H).
- 5,7-Dihydro-6H-dibenz[c,e]azepin-6-carbonitril und Cyclopentanol den 5,7-Dihydro-6H-dibenz[c,e]-azepin -6-carboximidsäure-cyclopentylester als Sirup, $^1$H-NMR($CDCl_3$): 1,5-2,1 (8H), 4,2 (s, 4H), 4,67 (breites s, NH), 5,1 (m, OCH), 7,3-7,7 (8H).

Beispiel 3

5 g 5,7-Dihydro-6H-dibenz[c,e]azepin-6-carbonitril in 60 ml absolutem Alkohol werden in Gegenwart von 30 mg Natriumäthylat während 1 Stunde auf Rückflusstemperatur erhitzt. Nach Abdestillieren des Alkohols wird der Rückstand mit Wasser versetzt und das Gemisch dreimal mit Methylenchloride extrahiert. Die vereinigten Extrakte werden mit Wasser gewaschen, über wasserfreiem Natriumsulfat getrocknet und eingedampft, und der Rückstand wird im Kugelrohrofen bei 230-250 ° C/0,1 mmHg destilliert. Man erhält den 5,7-Dihydro-6H-dibenz[c,e]azepin -6-carboximidsäure-äthylester als klares Harz, das fest wird; Smp. 68-71 ° C.

Beispiel 4

1,19 g 5,7-Dihydro-6H-dibenz[c,e]azepin-6-carboxamid werden in 10 ml Methylenchlorid gelöst, und die Lösung wird mit 1,14 g Triäthyloxonium-tetrafluoroborat versetzt und während 20 Stunden bei Raumtemperatur gerührt. Dann wird das Gemisch auf 10%ige Natriumcarbonatlösung/Eis gegossen und das Ganze mit Methylenchlorid extrahiert. Man wäscht die vereinigten Extrakte mit 10%iger Natriumcarbonatlösung, trocknet sie über wasserfreiem Natriumsulfat und dampft sie unter vermindertem Druck ein. Auf diese Weise erhält man den rohen 5,7-Dihydro-6H-dibenz[c,e]azepin -6-carboximidsäure-äthylester,der durch eine Filtration an Kieselgel [Laufmittel: n-Hexan/Aethylacetat (1:1)] gereinigt wird; Smp. 74-76 ° C (aus n-Hexan kristallisiert).

Beispiel 5

Zu einer Lösung von 1,0 g 5,7-Dihydro-6H-dibenz[c,e]azepin-6-carboximidsäure-äthylester in 8 ml Benzol wird eine Lösung von 0,68 g Oxalsäure in 6 ml Alkohol zugetropft. Man rührt die Lösung während weiterer 15 Minuten, destilliert anschliessend das Lösungsmittel ab und kristallisiert den Rückstand aus Aethanol/Diäthyläther. Nach Umkristallisieren aus wenig Aethanol erhält man weisse Kristalle von 5,7-Dihydro-6H-dibenz[c,e]azepin-6-carboximidsäure -äthylester-1,5-oxalat, Smp. 120 ° C (unter Zersetzung). [Die Addition von 1,5 Aequivalenten Oxalsäure ist durch Elementaranalyse des Produkts angezeigt.]

Beispiel 6

Zu einer eiskalten Lösung von 1 g 5,7-Dihydro-6H-dibenz[c,e]azepin-6-carboximidsäure-äthylester in Diäthyläther wird 1 ml konzentrierte Phosphorsäure zugetropft. Nach Abdestiliieren des Lösungsmittels wird das zuerst ölige Produkt aus Alkohol/Benzol kristallisiert, und man erhält nach Umkristallisieren aus wenig Alkohol weisse Kristalle von 5,7-Dihydro-6H-dibenz[c,e]azepin-6-carboximidsäure-äthylester-diphosphat, Smp. 154 ° C (unter Zersetzung). [Die Addition von 2 Aequivalenten Phosphorsäure ist durch Elementaranalyse des Produktes angezeigt.]

In analoger Weise erhält man ausgehend von 5,7-Dihydro-6H-dibenz[c,e]azepin-6-carboximidsdäure-äthylester und konzentrierter Schwefelsäure das 5,7-Dihydro-6H-dibenz[c,e]azepin-6-carboximidsäure-äthylester-sulfat als weisse Kristalle, Smp. 146 ° C (unter Zersetzung; aus Methanol kristallisiert).

11. Herstellung des Ausgangsmaterials der Formel II:

Beispiel 7

180 ml 50%ige Natronlauge werden bei ca. 60 ° C der Reihe nach mit 7,7 g Cyanamid, 1,3 g Tricaprylmethylammoniumchlorid, 360 ml Benzol und 62 g 2,2'-Bis(brommethyl)-biphenyl versetzt, und das Gemisch wird während 6 Stunden bei 60-70 ° C gerührt. Nach Erkalten des Reaktionsgemisches wird dieses in einen Scheidetrichter gegeben und mit 50 ml Methylenchlorid geschüttelt, wobei das ausgefallene Produkt in die Methylenchloridphase aufgenommen wird. Die wässrige Phase wird zweimal mit Methylenchlorid nachextrahiert. Anschliessend werden die vereinigten Extrakte mit Wasser gewaschen, über wasserfreiem Natriumsulfat getrocknet und eingedampft, und der Rückstand wird in 100 ml Toluol aufgenommen und die Lösung auf Rückflusstemperatur erhitzt, filtriert und bei ca. 50 ° C mit 200 ml tert.Butyl-methyl-äther versetzt. Schliesslich wird das durch Eiskühlung ausgefallene Kristallisat abgenutscht und getrocknet. Auf diese Weise erhält man reines 5,7-Dihydro-6H-dibenz[c,e]azepin-6-carbonitril, Smp. 121-124 ° C.

Beispiel 8

Zu einer auf 5 ° C abgekühlten Lösung von 7 g 6,7-Dihydro-5H-dibenz[c,e]azepin in 50 ml Chloroform lässt man eine Lösung von 1,91 g Bromcyan in 20 ml Chloroform innert 25 Minuten bei 5-10 ° C zutropfen. Man rührt das Reaktionsgemisch noch 1 Stunde und filtriert dann wenig Hydrobromidsalz des noch unreagierten Azepins ab. Das Filtrat wird dreimal mit Wasser gewaschen, über wasserfreiem Natriumsulfat getrocknet und eingedampft. Man erhält festes 5,7-Dihydro-6H-dibenz[c,e]azepin-6-carbonitril, Smp. 123-126 ° C.

Beispiel 9

Ein Gemisch von 1,19 g 5,7-Dihydro-6H-dibenz[c,e]azepin-6-carboxamid (s. Beispiel 10), 12 ml Chloroform, 2,7 ml 50%ige Natronlauge und 50 mg Triäthylamin wird während 1 Stunde bei Raumtemperatur gerührt. Dann wird das Reaktionsgemisch auf Eiswasser gegossen und das Ganze mit Methylenchlorid extrahiert. Die Extrakte werden mit Wasser gewaschen, über wasserfreiem Natriumsulfat getrocknet und eingedampft, und der Rückstand wird durch Flash-Chromatographie an Kieselgel [Laufmittel: n-Hexan/Aethylacetat (9:1)] gereinigt. Man erhält das 5,7-Dihydro-6H-dibenz[c,e]azepin-6-carbonitril, Smp. 118-121 °C.

III. Herstellung der Ausgangsmaterialien der Formel IV

Beispiel 10

Ein Gemisch von 1,95 g 6,7-Dihydro-5H-dibenz[c,e]azepin und 3 g Harnstoff wird während 15 Minuten bei 120 °C gerührt und dann auf Eiswasser gegossen. Nach Extrahieren des resultierenden wässrigen Gemisches mit Methylenchlorid werden die vereinigten Extrakte mit Wasser gewaschen, über wasserfreiem Natriumsulfat getrocknet und eingedampft. Man kristallisiert den festen Rückstand aus n-Hexan/Aethylacetat um und erhält auf diese Weise reines 5,7-Dihydro-6H-dibenz[c,e]azepin-6-carboxamid, Smp. 212-214 °C.

IV. Formulierungsbeispiele:

Beispiel 11

Ein Spritzpulver hat folgende Zusammensetzung:

Ein Spritzpulver hat folgende Zusammensetzung:

| | Gewichtsprozent |
|---|---|
| Verbindung der Formel I bzw. Säureadditionssalz davon (Wirkstoff) | 50 |
| Hydratisierte Kieselsäure (Trägerstoff) | 37 |
| Polycarbonsäure-Natriumsalz (Dispergator) | 4 |
| Nonylphenyl-(10)äthoxylat (Netzmittel) | 4 |
| Kaolin (Trägerstoff) | 5 |
| | 100 |

Der Wirkstoff wird mit dem Kaolin vermischt und separat das Netzmittel auf die hydratisierte Kieselsäure aufgezogen und der Dispergator zugesetzt. Dann wird das Ganze homogen vermischt und in einer geeigneten Mühle feingemahlen. Das nun vorliegende Spritzpulver wird durch Wasser spontan benetzt und ergibt auf diese Weise eine gebrauchsfertige Dispersion.

Beispiel 12

Ein emulgierbares Konzentrat hat folgende Zusammensetzung:

| | g/Liter |
|---|---|
| Verbindung der Formel I (Wirkstoff) | 250 |
| Polyarylphenol-(18)äthoxylat (Emulgator) | 300 |
| Isoterdecylalkohol (Antischaummittel) | 20 |
| Polyvinylpyrrolidon (Dispergator) | 20 |
| N-Methyl-pyrrolidon (Lösungsmittel) | ad 1000 ml |

Der Wirkstoff, der Emulgator und das Antischaummittel werden unter Rühren in das Lösungsmittel aufgenommen. Hierauf wird der Dispergator zugegeben und unter Rühren aufgelöst. Nach Verdünnen mit Wasser ergibt das so entstandene emulgierbare Konzentrat eine Emulsion, die sich als gebrauchsfertige Spritzbrühe gut eignet.

**Patentansprüche**
**Patentansprüche für folgende Vertragsstaaten : BE, CH, DE, FR, GB, IT, LI, NL, SE**

**1.** Verbindungen der allgemeinen Formel

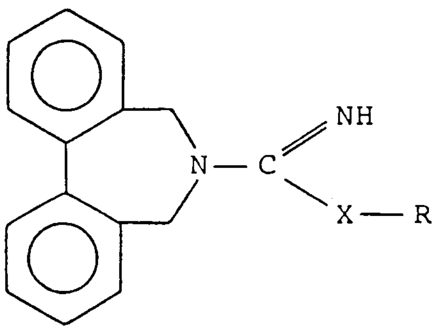

I

worin
R $C_{1-12}$-Alkyl, $C_{3-12}$-Alkenyl, $C_{3-12}$-Alkinyl oder $C_{3-8}$-Cycloalkyl und
X Sauerstoff oder Schwefel bedeuten,
und keine der allfälligen Mehrfachbindungen von R in $\alpha$-Stellung zu X steht, sowie Säureadditionssalze dieser Verbindungen.

**2.** Verbindungen nach Anspruch 1, dadurch gekennzeichnet, dass R $C_{1-3}$-Alkyl bedeutet.

**3.** Verbindungen nach Anspruch 1 oder 2, dadurch gekennzeichnet, dass X Sauerstoff bedeutet.

**4.** 5,7-Dihydro-6H-dibenz[c,e]azepin-6-carboximidsäure-äthylester und dessen Hydrochlorid gemäß Anspruch 3.

**5.** 5,7-Dihydro-6H-dibenz[c,e]azepin-6-carboximidsäure-methylester und dessen Hydrochlorid gemäß Anspruch 3.

**6.** 5,7-Dihydro-6H-dibenz[c,e]azepin-6-carboximidsäure-propylester und dessen Hydrochlorid gemäß Anspruch 3.

**7.** 5,7-Dihydro-6H-dibenz[c,e]azepin-6-carboximidsäure-isopropylester und dessen Hydrochlorid.

**8.** Eine Verbindung nach Anspruch 1, ausgewählt aus:
5,7-Dihydro-6H-dibenz[c,e]azepin-6-thiocarboximidsäure-äthylester und dessen Hydrochlorid,

5,7-Dihydro-6H-dibenz[c,e]azepin-6-thiocarboximidsäure-dodecylester,
5,7-Dihydro-6H-dibenz[c,e]azepin-6-thiocarboximidsäure-2-butylester,
5,7-Dihydro-6H-dibenz[c,e]azepin-6-carboximidsäure-allylester und dessen Hydrochlorid,
5,7-Dihydro-6H-dibenz[c,e]azepin-6-carboximidsäure-butylester,
5,7-Dihydro-6H-dibenz[c,e]azepin-6-carboximidsäure-2-butylester,
5,7-Dihydro-6H-dibenz[c,e]azepin-6-carboximidsäure-isobutylester,
5,7-Dihydro-6H-dibenz[c,e]azepin-6-carboximidsäure-octylester,
5,7-Dihydro-6H-dibenz[c,e]azepin-6-carboximidsäure-2-propinylester,
5,7-Dihydro-6H-dibenz[c,e]azepin-6-carboximidsäure-cyclohexylester,
5,7-Dihydro-6H-dibenz[c,e]azepin-6-carboximidsäure-cyclopentylester,
5,7-Dihydro-6H-dibenz[c,e]azepin-6-carboximidsäure-äthylester-1,5 oxalat und
5,7-Dihydro-6H-dibenz[c,e]azepin-6-carboximidsäure-äthylester-diphosphat.

**9.** 5,7-Dihydro-6H-dibenz[c,e]azepin-6-carbonitril.

**10.** Verbindungen der allgemeinen Formel

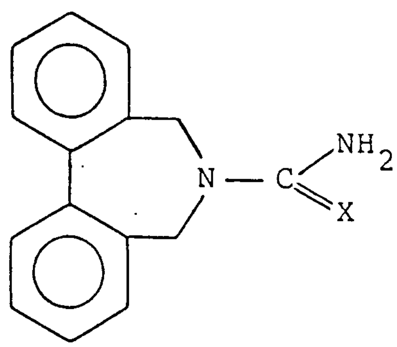

IV

worin X Sauerstoff oder Schwefel bedeutet.

**11.** 5,7-Dihydro-6H-dibenz[c,e]azepin-6-carboxamid gemäß Anspruch 10.

**12.** Schädlingsbekämpfungsmittel, dadurch gekennzeichnet, dass es eine wirksame Menge mindestens einer Verbindung der allgemeinen Formel

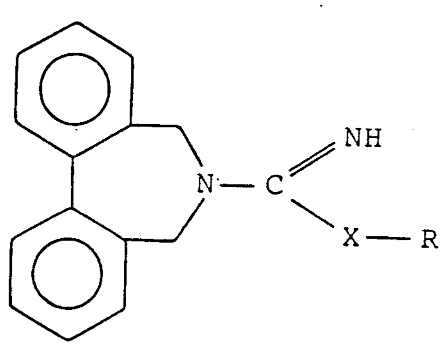

I

worin
R $C_{1-12}$-Alkyl, $C_{3-12}$-Alkenyl, $C_{3-12}$-Alkinyl oder $C_{3-8}$-Cycloalkyl und
X Sauerstoff oder Schwefel bedeuten,
und keine der allfälligen Mehrfachbindungen von R in $\alpha$-Stellung zu X steht,
oder eines Säureadditionssalzes davon, sowie Formulierungshilfsstoffe enthält.

**13.** Schädlingsbekämpfungsmittel nach Anspruch 12, dadurch gekennzeichnet, dass es eine wirksame Menge mindestens einer aus der Gruppe
5,7-Dihydro-6H-dibenz[c,e]azepin-6-carboximidsäure-äthylesterund dessen Hydrochlorid,
5,7-Dihydro-6H-dibenz[c,e]azepin-6-carboximidsäure-methylesterund dessen Hydrochlorid,
5,7-Dihydro-6H-dibenz[c,e]azepin-6-carboximidsäure-propylesterund dessen Hydrochlorid und
5,7-Dihydro-6H-dibenz(c,e]azepin-6-carboximidsäure-iso-propylester und dessen Hydrochlorid
ausgewählten Verbindung sowie Formulierungshilfsstoffe enthält.

**14.** Verfahren zur Herstellung von Verbindungen der allgemeinen Formel

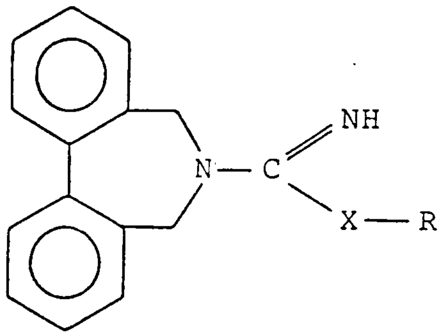

I

worin
R $C_{1-12}$-Alkyl, $C_{3-12}$-Alkenyl, $C_{3-12}$-Alkinyl oder $C_{3-8}$-Cycloalkyl
und
X Sauerstoff oder Schwefel bedeuten,
und keine der allfälligen Mehrfachbindungen von R in $\alpha$-Stellung zu X steht,
und von ihren Säureadditionssalzen, dadurch gekennzeichnet, dass man
5,7-Dihydro-6H-dibenz[c,e]azepin-6-carbonitril mit einem Alkohol bzw. Thiol der allgemeinen Formel

R-XH III

worin R und X die oben angegebenen Bedeutungen besitzen, oder mit einem Alkalimetallsalz davon umsetzt, oder
und erwunschtenfalls eine erhaltene Verbindung der Formel I durch Umsetzung mit einer Säure in das entsprechende Säureadditionssalz überführt.

**15.** Verfahren zur Bekämpfung von Schädlingen, dadurch gekennzeichnet, dass man das zu schützende Gut oder die Schädlingen selbst mit einer wirksamen Menge einer Verbindung gemäss einem der Ansprüche 1 bis 8 bzw. eines Mittels gemäss Anspruch 12 oder 13 behandelt.

**16.** Verwendung einer Verbindung gemäss einem der Ansprüche 1 bis 8 bzw. eines Mittels gemäss Anspruch 12 oder 13 zur Bekämpfung von Schädlingen.

**17.** Schädlingsbekämpfungsmittel nach Anspruch 12, dadurch gekennzeichnet, dass es als zusätzlichen Wirkstoff ein Akarizid zur Bekämpfung des unentwickelten Stadiums der Milben, also der Eier und Larven, enthält.

**18.** Verfahren zur Herstellung von Verbindungen der allgemeinen Formel

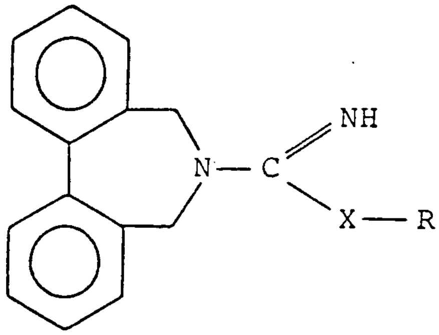

I

worin
R Methyl oder Aethyl
und

X Sauerstoff oder Schwefel bedeuten,
und von ihren Säureadditionssalzen, dadurch gekennzeichnet, dass man einen Harnstoff bzw. Thioharnstoff der allgemeinen Formel

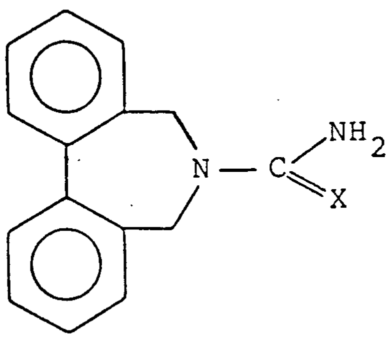

IV

worin X die oben angegebene Bedeutung besitzt, alkyliert,
und erwunschtenfalls eine erhaltene Verbindung der Formel I durch Umsetzung mit einer Säure in das entsprechende Säureadditionssalz überführt.

**Patentansprüche für folgenden Vertragsstaat : AT**

**1.** Schädlingbekämpfungsmittel, dadurch gekennzeichnet, dass es eine wirksame Menge mindestens einer Verbindung der allgemeinen Formel

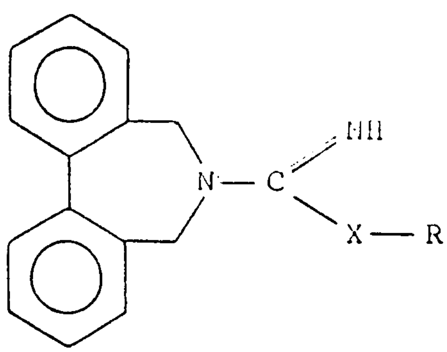

I

worin
R $C_{1-12}$-Alkyl, $C_{3-12}$-Alkenyl, $C_{3-12}$-Alkinyl oder $C_{3-8}$-Cycloalkyl

und

X Sauerstoff oder Schwefel bedeuten,

und keine der allfälligen Mehrfachbindungen von R in α-Stellung zu X steht, oder eines Säureadditionssalzes davon, sowie Formulierungshilfsstoffe enthält.

**2.** Schädlingsbekämpfungsmittel nach Anspruch 1, worin R $C_{1-3}$-Alkyl bedeutet.

**3.** Schädlingsbekämpfungsmittel nach Anspruch 1 oder 2, worin X Sauerstoff bedeutet.

**4.** Schädlingsbekämpfungsmittel nach Anspruch 1, dadurch gekennzeichnet, dass es eine wirksame Menge von 5,7-Dihydro-6H-dibenz[c,e]azepin-6-carboximidsäure-äthylester und/oder dessen Hydrochlorid sowie Formulierungshilfsstoffe enthält.

**5.** Schädlingsbekämpfungsmittel nach Anspruch 1, dadurch gekennzeichnet, dass es eine wirksame Menge von 5,7-Dihydro-6H-dibenz[c,e]azepin-6-carboximidsäure-methylester und/oder dessen Hydrochlorid sowie Formulierungshilfsstoffe enthält.

**6.** Schädlingsbekämpfungsmittel nach Anspruch 1, dadurch gekennzeichnet, dass es eine wirksame Menge von 5,7-Dihydro-6H-dibenz[c,e]azepin-6-carboximidsäure-propylester und/oder dessen Hydrochlorid sowie Formulierungshilfsstoffe enthält.

**7.** Schädlingsbekämpfungsmittel nach Anspruch 1, dadurch gekennzeichnet, dass es eine wirksame Menge von 5,7-Dihydro-6H-dibenz[c,e]azepin-6-carboximidsäure-isopropylester und/oder dessen Hydrochlorid sowie Formulierungshilfsstoffe enthält.

**8.** Schädlingsbekämpfungsmittel nach Anspruch 1, dadurch gekennzeichnet, dass es eine wirksame Menge mindestens einer aus der Gruppe

5,7-Dihydro-6H-dibenz[c,e]azepin-6-thiocarboximidsäure-äthylester und dessen Hydrochlorid,
5,7-Dihydro-6H-dibenz[c,e]azepin-6-thiocarboximidsäure-dodecylester,
5,7-Dihydro-6H-dibenz[c,e]azepin-6-thiocarboximidsäure-2-butylester,
5,7-Dihydro-6H-dibenz[c,e]azepin-6-carboximidsäure-allylester und dessen Hydrochlorid,
5,7-Dihydro-6H-dibenz[c,e]azepin-6-carboximidsäure-butylester,
5,7-Dihydro-6H-dibenz[c,e]azepin-6-carboximidsäure-2-butylester,
5,7-Dihydro-6H-dibenz[c,e]azepin-6-carboximidsäure-isobutylester,
5,7-Dihydro-6H-dibenz[c,e]azepin-6-carboximidsäure-octylester,
5,7-Dihydro-6H-dibenz[c,e]azepin-6-carboximidsäure-2-propinylester,
5,7-Dihydro-6H-dibenz[c,e]azepin-6-carboximidsäure-cyclohexylester,
5,7-Dihydro-6H-dibenz[c,e]azepin-6-carboximidsäure-cyclopentylester,
5,7-Dihydro-6H-dibenz[c,e]azepin-6-carboximidsäure-äthylester-1,5 oxalat und
5,7-Dihydro-6H-dibenz[c,e]azepin-6-carboximidsäure-äthylester-diphosphat

ausgewählten Verbindung sowie Formulierungshilfsstoffe enthält.

**9.** Verfahren zur Herstellung von Verbindungen der allgemeinen Formel

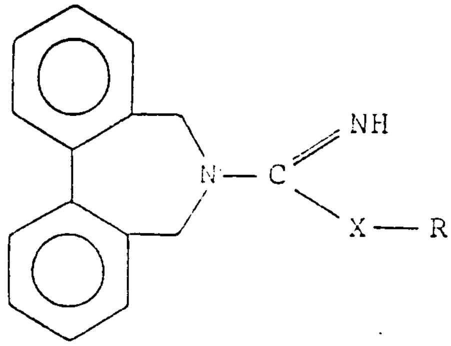

I

worin

R $C_{1-2}$-Alkyl, $C_{3-2}$-Alkenyl, $C_{3-2}$-Alkinyl oder $c_{3-8}$-Cycloalkyl und

X Sauerstoff oder Schwefel bedeuten,

und keine der allfälligen Mehrfachbindungen von R in α-Stellung zu X steht, und von ihren Säureadditionssalzen, dadurch gekennzeichnet, dass man 5,7-Dihydro-6H-dibenz[c,e]azepin-6-carbonitril mit einem Alkohol bzw. Thiol der allgemeinen Formel

R-XH III

worin R und X die oben angegebenen Bedeutungen besitzen, oder mit einem Alkalimetallsalz davon umsetzt und erwünschtenfalls eine erhaltene Verbindung der Formel I durch Umsetzung mit einer Säure in das entsprechende Säureadditionssalz überführt.

**10.** Verfahren zur Herstellung eines Schädlingsbekämpfungsmittels, dadurch gekennzeichnet, dass man mindestens eine der in Anspruch 1 genannten Verbindungen mit Formulierungshilfsstoffen vermischt.

**11.** Verfahren zur Bekämpfung von Schädlingen, dadurch gekennzeichnet, dass man das zu schützende Gut oder die Schädlinge selbst mit einer wirksamen Menge mindestens einer in einem der Ansprüche 1 bis 8 genannten Verbindung bzw. eines Mittels gemäss einem dieser Ansprüche behandelt.

**12.** Verwendung einer in einem der Ansprüche 1 bis 8 genannten Verbindung bzw. eines Mittels gemäss einem dieser Ansprüche zur Bekämpfung von Schädlingen.

**13.** Schädlingsbekämpfungsmittel nach Anspruch 1, dadurch gekennzeichnet, dass es als zusätzlichen Wirkstoff ein Akarizid zur Bekämpfung des unentwickelten Stadiums der Milben, also der Eier und Larven, enthält.

**14.** Verfahren zur Herstellung von Verbindungen der allgemeinen Formel

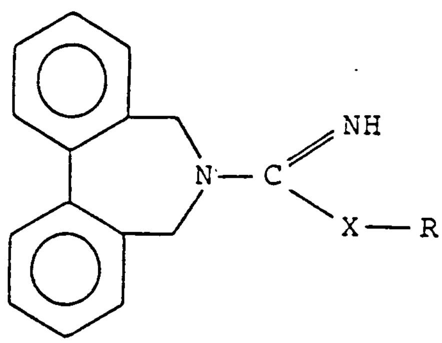

I

worin

R Methyl oder Aethyl und

X Sauerstoff oder Schwefel bedeuten,

und von ihren Säureadditionssalzen, dadurch gekennzeichnet, dass man einen Harnstoff bzw. Thioharnstoff der allgemeinen Formel

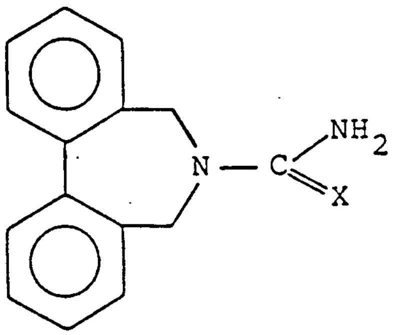

IV

worin X die oben angegebene Bedeutung besitzt, alkyliert, und erwünschtenfalls eine erhaltene Verbindung der Formel I durch Umsetzung mit einer Säure in das entsprechende Säureadditionssalz überführt.

**Claims**

**Claims for the following Contracting States : BE, CH, DE, FR, GB, IT, LI, NL, SE**

**1.** A compound of the general formula

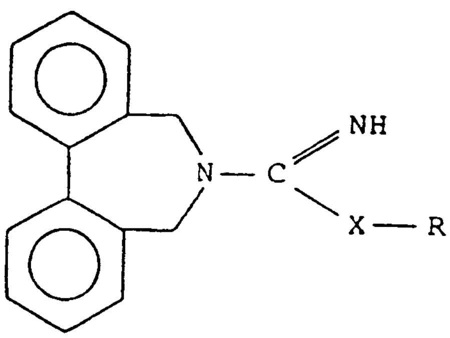

I

wherein

R is $C_{1-12}$alkyl, $C_{3-12}$alkenyl, $C_{3-12}$alkynyl or $C_{3-8}$-cycloalkyl and

X is oxygen or sulphur,

and none of the possible multiple bonds of R is present in the $\alpha$-position to X, or an acid addition salt of such a compound.

**2.** A compound according to claim 1, wherein R is $C_{1-3}$-alkyl.

**3.** A compound according to claim 1 or 2, wherein X is oxygen.

**4.** Ethyl 5,7-dihydro-6H-dibenz[c,e]azepine-6-carboximidate and its hydrochloride according to claim 3.

**5.** Methyl 5,7-dihydro-6H-dibenz[c,e]azepine-6-carboximidate and its hydrochloride according to claim 3.

**6.** Propyl 5,7-dihydro-6H-dibenz[c,e]azepine-6-carboximidate and its hydrochloride according to claim 3.

**7.** Isopropyl 5,7-dihydro-6H-dibenz[c,e]azepine-6-carboximidate and its hydrochloride.

**8.** A compound according to claim 1, selected from:
ethyl 5,7-dihydro-6H-dibenz[c,e]azepine-6-thiocarboximidate and its hydrochloride,
dodecyl 5,7-dihydro-6H-dibenz[c,e]azepine-6-thiocarboximidate,
2-butyl 5,7-dihydro-6H-dibenz[c,e]azepine-6-thiocarboximidate,
allyl 5,7-dihydro-6H-dibenz[c,e]azepine-6-carboximidate and its hydrochloride,
butyl 5,7-dihydro-6H-dibenz[c,e]azepine-6-carboximidate,

2-butyl 5,7-dihydro-6H-dibenz[c,e]azepine-6-carboximidate,
isobutyl 5,7-dihydro-6H-dibenz[c,e]azepine-6-carboximidate,
octyl 5,7-dihydro-6H-dibenz[c,e]azepine-6-carboximidate,
2-propynyl 5,7-dihydro-6H-dibenz[c,e]azepine-6-carboximidate,
cyclohexyl 5,7-dihydro-6H-dibenz[c,e]azepine-6-carboximidate,
cyclopentyl 5,7-dihydro-6H-dibenz[c,e]azepine-6-carboximidate,
ethyl 5,7-dihydro-6H-dibenz(c,e]azepine-6-carboximidate 1.5-oxalate and
ethyl 5,7-dihydro-6H-dibenz[c,e]azepine-6-carboximidate diphosphate.

**9.** 5,7-Dihydro-6H-dibenz[c,e)azepine-6-carbonitrile.

**10.** A compound of the general formula

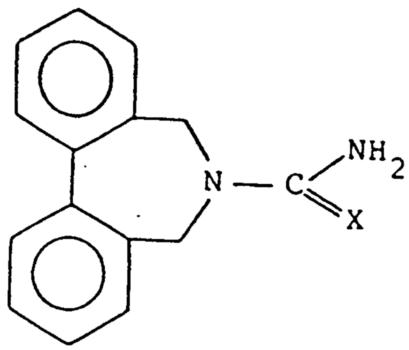

IV

wherein X is oxygen or sulphur.

**11.** 5,7-Dihydro-6H-dibenz[c,e]azepine-6-carboxamide according to claim 10.

**12.** A pest control composition which comprises an effective amount of at least one compound of the general formula

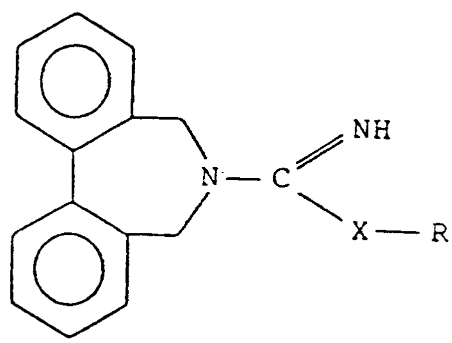

I

wherein
R is $C_{1-12}$alkyl, $C_{3-12}$alkenyl, $C_{3-12}$alkynyl or $C_{3-8}$-cycloalkyl and
X is oxygen or sulphur,
and none of the possible multiple bonds of R is present in the $\alpha$-position to X,
or an acid addition salt thereof, as well as formulation adjuvants.

**13.** A pest control composition according to claim 12, which comprises an effective amount of at least one compound selected from the group
ethyl 5,7-dihydro-6H-dibenz[c,e]azepine-6-carboximidate and its hydrochloride,
methyl 5,7-dihydro-6H-dibenz[c,e]azepine-6-carboximidate and its hydrochloride,

propyl 5,7-dihydro-6H-dibenz[c,e]azepine-6-carboximidate and its hydrochloride and isopropyl 5,7-dihydro-6H-dibenz[c,e]azepine-6-carboximidate and its hydrochloride, as well as formulation adjuvants.

**14.** A process for the manufacture of a compound of the general formula

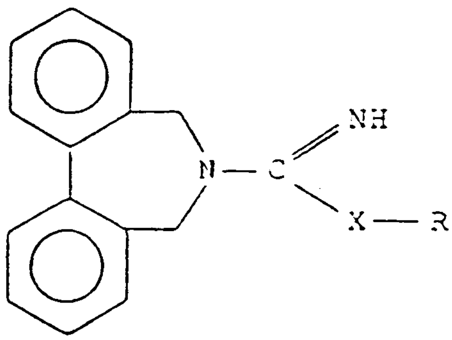

I

wherein
R is $C_{1-12}$alkyl, $C_{3-12}$alkenyl, $C_{3-12}$alkynyl or $C_{3-8}$-cycloalkyl and
X is oxygen or sulphur,
and none of the possible multiple bonds of R is present in the α-position to X,
or of an acid addition salt thereof, which process comprises
reacting 5,7-dihydro-6H-dibenz[c,e]azepine-6-carbonitrile with an alcohol or thiol of the general formula

R-XH III

wherein R and X are as defined above,
or with an alkali metal salt thereof, and, if desired, converting a resulting compound of formula I into the corresponding acid addition salt by reaction with an acid.

**15.** A method for the control of pests, which method comprises treating the locus to be protected or the pests themselves with an effective amount of a compound in accordance with any one of claims 1 to 8 or of a composition in accordance with claim 12 or 13.

**16.** The use of a compound in accordance with any one of claims 1 to 8 or of a composition in accordance with claim 12 or 13 for the control of pests.

**17.** A pest control composition according to claim 12, which comprises as an additional active substance an acaricide for the control of undeveloped stages of mites, namely of the eggs and larvae.

**18.** A process for the manufacture of a compound of the general formula

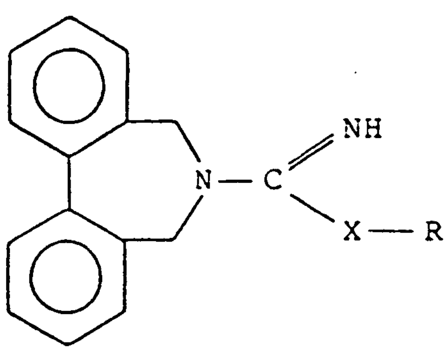

I

wherein
R is methyl or ethyl and
X is oxygen or sulphur,
or of an acid addition salt thereof, which process comprises alkylating a urea or thiourea of the general formula

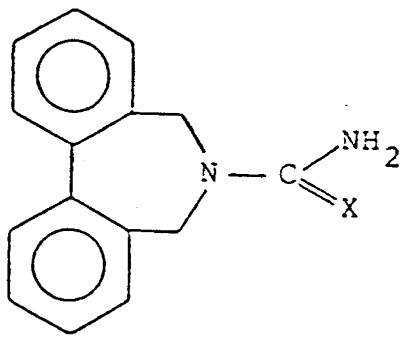

IV

wherein X is as defined above,
and, if desired, converting a resulting compound of formula I into the corresponding acid addition salt by reaction with an acid.

**Claims for the following Contracting State : AT**

1. A pest control composition which comprises an effective amount of at least one compound of the general formula

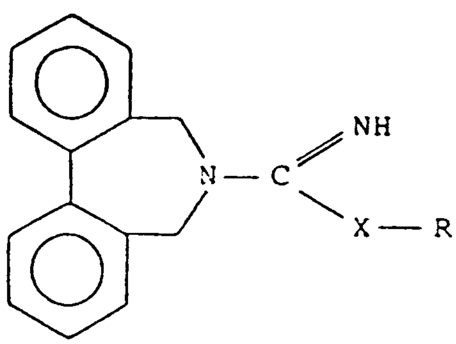

I

wherein
R is $C_{1-12}$alkyl, $C_{3-12}$alkenyl, $C_{3-12}$alkynyl or $C_{3-8}$-cycloalkyl and
X is oxygen or sulphur,
and none of the possible multiple bonds of R is present in the $\alpha$-position to X,
or an acid addition salt thereof, as well as formulation adjuvants.

2. A pest control composition according to claim 1, wherein R is $C_{1-3}$alkyl.

3. A pest control composition according to claim 1 or 2, wherein X is oxygen.

4. A pest control composition according to claim 1, which comprises an effective amount of ethyl 5,7-dihydro-6H-dibenz[c,e]azepine-6-carboximidate and/or its hydrochloride, as well as formulation adjuvants.

5. A pest control composition according to claim 1, which comprises an effective amount of methyl 5,7-dihydro-6H-dibenz[c,e]azepine-6-carboximidate and/or its hydrochloride, as well as formulation adjuvants.

6. A pest control composition according to claim 1, which comprises an effective amount of propyl 5,7-dihydro-6H-dibenz[c,e]azepine-6-carboximidate and/or its hydrochloride, as well as formulation adjuvants.

7. A pest control composition according to claim 1, which comprises an effective amount of isopropyl 5,7-dihydro-6H-dibenz[c,e]azepine-6-carboximidate and/or its hydrochloride, as well as formulation adjuvants.

8. A pest control composition according to claim 1, which comprises an effective amount of at least one compound selected from the group:
ethyl 5,7-dihydro-6H-dibenz[c,e]azepine-6-thiocarboximidate and its hydrochloride,
dodecyl 5,7-dihydro-6H-dibenz[c,e]azepine-6-thiocarboximidate,
2-butyl 5,7-dihydro-6H-dibenz[c,e]azepine-6-thiocarboximidate,
allyl 5,7-dihydro-6H-dibenz[c,e]azepine-6-carboximidate and its hydrochloride,
butyl 5,7-dihydro-6H-dibenz[c,e]azepine-6-carboximidate,
2-butyl 5,7-dihydro-6H-dibenz[c,e]azepine-6-carboximidate,
isobutyl 5,7-dihydro-6H-dibenz[c,e]azepine-6-carboximidate,
octyl 5,7-dihydro-6H-dibenz[c,e]azepine-6-carboximidate,
2-propynyl 5,7-dihydro-6H-dibenz[c,e]azepine-6-carboximidate,
cyclohexyl 5,7-dihydro-6H-dibenz[c,e]azepine-6-carboximidate,
cyclopentyl 5,7-dihydro-6H-dibenz[c,e]azepine-6-carboximidate,
ethyl 5,7-dihydro-6H-dibenz[c,e]azepine-6-carboximidate 1.5-oxalate and
ethyl 5,7-dihydro-6H-dibenz[c,e]azepine-6-carboximidate diphosphate,
as well as formulation adjuvants.

9. A process for the manufacture of a compound of the general formula

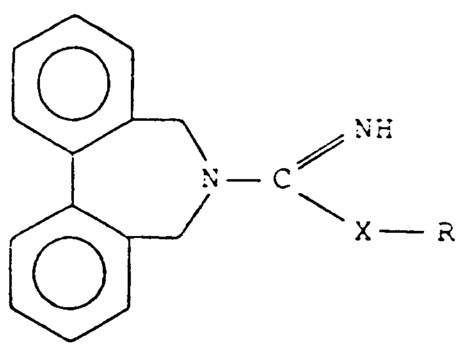

I

wherein
R is $C_{1-12}$alkyl, $C_{3-12}$alkenyl, $C_{3-12}$alkynyl or $C_{3-8}$-cycloalkyl and
X is oxygen or sulphur,
and none of the possible multiple bonds of R is present in the $\alpha$-position to X,
or of an acid addition salt thereof, which process comprises
reacting 5,7-dihydro-6H-dibenz[c,e]azepine-6-carbonitrile with an alcohol or thiol of the general formula

R-XH III

wherein R and X are as defined above,
or with an alkali metal salt thereof, and, if desired, converting a resulting compound of formula I into the corresponding acid addition salt by reaction with an acid.

10. A process for the manufacture of a pest control composition, which comprises mixing at least one of the compounds mentioned in claim 1 with formulation adjuvants.

11. A method for the control of pests, which method comprises treating the locus to be protected or the

pests themselves with an effective amount of at least one compound mentioned in any one of claims 1 to 8 or of a composition in accordance with any one of those claims.

**12.** The use of a compound mentioned in any one of claims 1 to 8 or of a composition in accordance with any one of those claims for the control of pests.

**13.** A pest control composition according to claim 1, which comprises as an additional active substance an acaricide for the control of undeveloped stages of mites, namely of the eggs and larvae.

**14.** A process for the manufacture of a compound of the general formula

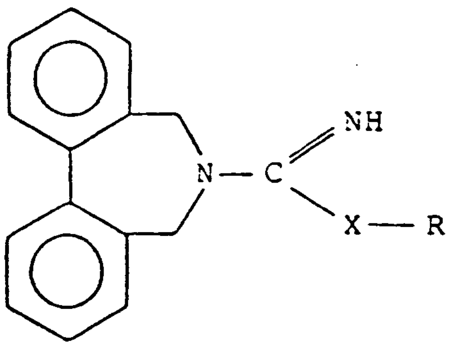

I

wherein
R is methyl or ethyl and
X is oxygen or sulphur,
or of an acid addition salt thereof, which process comprises alkylating a urea or thiourea of the general formula

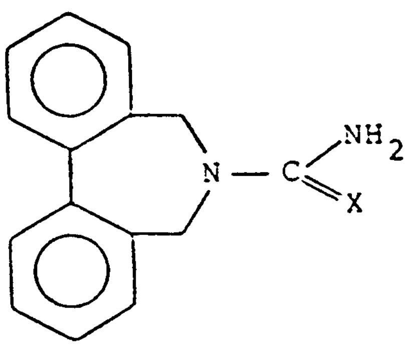

IV

wherein X is as defined above,
and, if desired, converting a resulting compound of formula I into the corresponding acid addition salt by reaction with an acid.

**Revendications**
**Revendications pour les Etats contractants suivants : BE, CH, DE, FR, GB, IT, LI, NL, SE**

**1.** Composés de formule générale

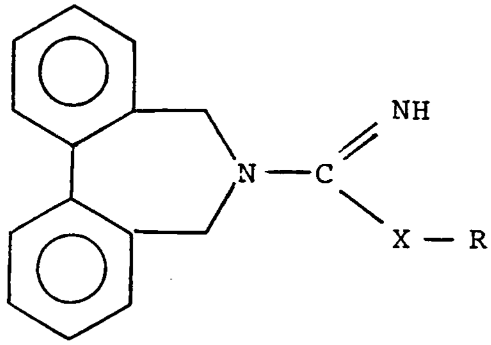

I

dans laquelle

R représente un groupe alkyle en C 1-C 12, alcényle en C 3-C 12, alcynyle en C 3-C 12 ou cycloalkyle en C 3-C 8, et

X représente l'oxygène ou le soufre,

aucune des liaisons multiples éventuelles de R ne devant être en position alpha par rapport à X,

et les sels de ces composés formés par addition avec des acides.

**2.** Composés selon revendication 1, caractérisés en ce que R représente un groupe alkyle en C 1-C 3.

**3.** Composés selon revendication 1 ou 2, caractérisés en ce que X représente l'oxygène.

**4.** Le 5,7-dihydro-6H-dibenzo[c,e]azépine-6-carboximidate d'éthyle et son chlorhydrate selon revendication 3.

**5.** Le 5,7-dihydro-6H-dibenzo[c,e]azépine-6-carboximidate de méthyle et son chlorhydrate selon revendication 3.

**6.** Le 5,7-dihydro-6H-dibenzo[c,e]azépine-6-carboximidate de propyle et son chlorhydrate selon revendication 3.

**7.** Le 5,7-dihydro-6H-dibenzo[c,e] azépine-6-carboximidate d'isopropyle et son chlorhydrate.

**8.** Un composé selon revendication 1, choisi parmi les suivants :

le 5,7-dihydro-6H-dibenzo[c,e]azépine-6-thiocarboximidate d'éthyle et son chlorhydrate,
le 5,7-diydro-6H-dibenzo[c,e]azépine-6-thiocarboximidate de dodécyle,
le 5,7-dihydro-6H-dibenzo[c,e]azépine-6-thiocarboximidate de 2-butyle,
le 5,7-dihydro-6H-dibenzo[c,e]azépine-6-carboximidate d'allyle et son chlorhydrate,
le 5,7-dihydro-6H-dibenzo[c,e]azépine-6-carboximidate de butyle,
le 5,7-dihydro-6H-dibenzo[c,e]azépine-6-carboximidate de 2-butyle,
le 5,7-dihydro-6H-dibenzo[c,e]azépine-6-carboximidate d'isobutyle;
le 5,7-dihydro-6H-dibenzo[c,e]azépine-6-carboximidate d'octyle,
le 5,7-dihydro-6H-dibenzo[c,e]azépine-6-carboximidate de 2-propynyle,
le 5,7-dihydro-6H-dibenzo[c,e]azépine-6-carboximidate de cyclohexyle,
le 5,7-dihydro-6H-dibenzo[c,e]azépine-6-carboximidate de cyclopentyle,
le 5,7-dihydro-6H-dibenzo[c,e]azépine-6-carboximidate d'éthyle, 1,5-oxalate, et
le 5,7-dihydro-6H-dibenzo[c,e]azépine-6-carboximidate d'éthyle, diphosphate.

**9.** Le 5,7-dihydro-6H-dibenzo[c,e]azépine-6-carbonitrile.

**10.** Composés de formule générale

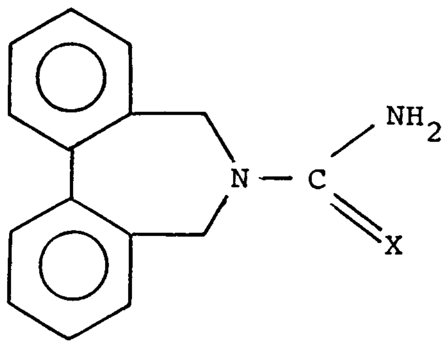

IV

dans laquelle X représente l'oxygène ou le soufre.

**11.** Le 5,7-dihydro-6H-dibenzo[c,e]azépine-6-carboxamide selon revendication 10.

**12.** Produit pesticide, caractérisé en ce qu'il contient une quantité efficace d'au moins un composé de formule générale

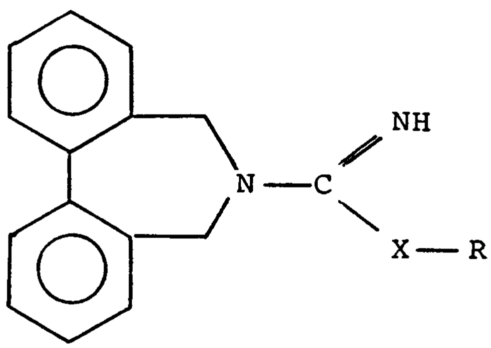

I

dans laquelle

R représente un groupe alkyle en C 1-C 12, alcényle en C 3-C 12, alcynyle en C 3-C 12 ou cycloalkyle en C 3-C 8, et

X représente l'oxygène ou le soufre,

aucune des liaisons multiples éventuelles de R ne devant se trouver en position alpha par rapport à X, ou un sel d'un tel composé formé par addition avec un acide, avec des produits auxiliaires de formulation.

**13.** Produit pesticide selon revendication 12, caractérisé en ce qu'il contient une quantité efficace d'au moins un composé choisi dans le groupe suivant :

5,7-dihydro-6H-dibenzo[c,e]azépine-6-carboximidate d'éthyle et son chlorhydrate,
5,7-dihydro-6H-dibenzo[c,e]azépine-6-carboximidate de méthyle et son chlorhydrate,
5,7-dihydro-6H-dibenzo[c,e]azépine-6-carboxymidate de propyle et son chlorhydrate,
5,7-dihydro-6H-dibenzo[c,e]azépine-6-carboximidate d'isopropyle et son chlorhydrate,

avec des produits auxiliaires de formulation.

**14.** Procédé de préparation des composés de formule générale

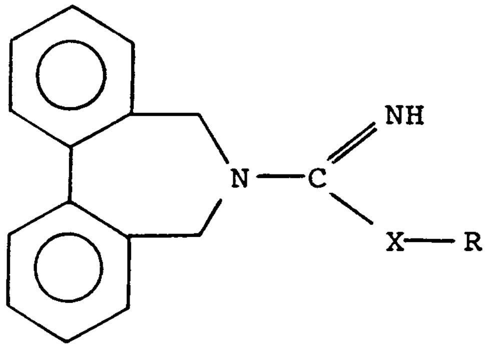

I

dans laquelle

R représente un groupe alkyle en C 1-C 12, alcényle en C 3-C 12, alcynyle en C 3-C 12 ou cycloalkyle en C 3-C 8, et

X représente l'oxygène ou le soufre,

aucune des liaisons multiples éventuelles de R ne devant se trouver en position alpha par rapport à X, et de leurs sels formés par addition avec des acides, caractérisé en ce que :

on fait réagir le 5,7-dihydro-6H-dibenzo[c,e] azépine-6-carbonitrile avec un alcool ou thiol respectivement de formule générale

R-XH III

dans laquelle R et X ont les significations indiquées ci-dessus, ou avec un sel de métal alcalin d'un tel alcool ou thiol,

et si on le désire, on convertit un composé ainsi obtenu, répondant à la formule I, en un sel formé par addition avec un acide par réaction avec l'acide correspondant.

**15.** Procédé pour combattre les parasites, caractérisé en ce que l'on traite l'objet à protéger ou les parasites eux-mêmes par une quantité efficace d'un composé selon l'une des revendications 1 à 8 ou d'un produit selon revendication 12 ou 13.

**16.** Utilisation d'un composé selon une des revendications 1 à 8 ou d'un produit selon revendication 12 ou 13, pour la lutte contre les parasites.

**17.** Produit pesticide selon revendication 12, caractérisé en ce qu'il contient une autre substance active consistant en un acaricide servant à combattre les acariens non développés, c'est-à-dire les oeufs et les larves.

**18.** Procédé de préparation des composés de formule générale

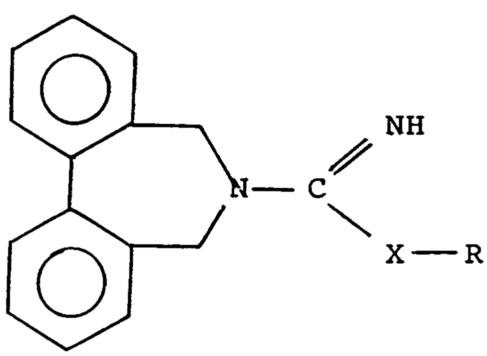

I

dans laquelle

R représente un groupe méthyle ou éthyle, et

X représente l'oxygène ou le soufre,

et de leurs sels formés par addition avec des acides, caractérisé en ce que l'on alkyle une urée ou thiourée respectivement de formule générale

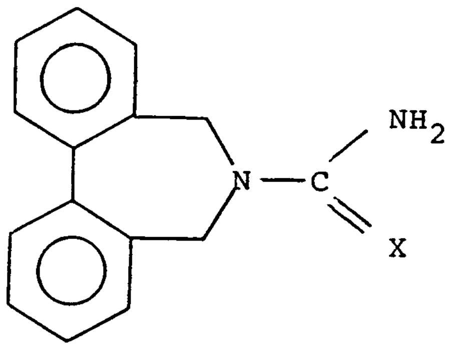

IV

dans laquelle X a les significations indiquées ci-dessus,

et si on le désire, on convertit un composé ainsi obtenu, répondant à la formule I, en sel formé par addition avec un acide, par réaction avec l'acide correspondant.

**Revendications pour l'Etat contractant suivant : AT**

1. Produit pesticide caractérisé en ce qu'il contient une quantité efficace d'au moins un composé de formule générale

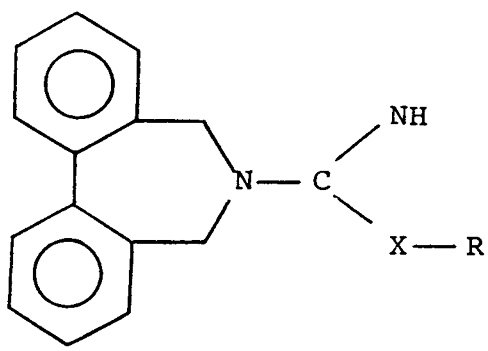

I

dans laquelle

R représente un groupe alkyle en C 1-C 12, alcényle en C 3-C 12, alcynyle en C 3-C 12 ou cycloalkyle en C 3-C 8, et

X représente l'oxygène ou le soufre,

aucune des liaisons multiples éventuelles de R ne devant se trouver en position alpha par rapport à X,

ou d'un sel d'un tel composé formé par addition avec un acide, avec des produits auxiliaires de formulation.

2. Produit pesticide selon revendication 1, pour lequel R est un groupe alkyle en C 1-C 3.

3. Produit pesticide selon revendication 1 ou 2, pour lequel X représente l'oxygène.

4. Produit pesticide selon revendication 1, caractérisé en ce qu'il contient une quantité efficace de 5,7-dihydro-6H-dibenzo[c,e]azépine-6-carboximidate d'éthyle et/ou de son chlorhydrate avec des produits auxiliaires de formulation.

5. Produit pesticide selon revendication 1, caractérisé en ce qu'il contient une quantité efficace de 5,7-dihydro-6H-dibenzo[c,e]azépine-6-carboximidate de méthyle et/ou de son chlorhydrate avec des produits auxiliaires de formulation.

6. Produit pesticide selon revendication 1, caractérisé en ce qu'il contient une quantité efficace de 5,7-dihydro-6H-dibenzo[c,e]azépine-6-carboximidate de propyle et/ou de son chlorhydrate avec des produits auxiliaires de formulation.

7. Produit pesticide selon revendication 1, caractérisé en ce qu'il contient une quantité efficace de 5,7-dihydro-6H-dibenzo[c,e]azépine-6-carboximidate d'isopropyle et/ou de son chlorhydrate avec des produits auxiliaires de formulation.

8. Produit pesticide selon revendication 1, caractérisé en ce qu'il contient une quantité efficace d'au moins un composé du groupe suivant :
le 5,7-dihydro-6H-dibenzo[c,e]azépine-6-thiocarboximidate d'éthyle et son chlorhydrate,
le 5,7-dihydro-6H-dibenzo[c,e]azépine-6-thiocarboximidate de dodécyle,
le 5,7-dihydro-6H-dibenzo[c,e]azépine-6-thiocarboximidate de 2-butyle,
le 5,7-dihydro-6H-dibenzo[c,e]azépine-6-carboximidate d'allyle et son chlorhydrate,
le 5,7-dihydro-6H-dibenzo[c,e]azépine-6-carboximidate de butyle,
le 5,7-dihydro-6H-dibenzo[c,e]azépine-6-carboximidate de 2-butyle,
le 5,7-dihydro-6H-dibenzo[c,e]azépine-6-carboximidate d'isobutyle;
le 5,7-dihydro-6H-dibenzo[c,e]azépine-6-carboximidate d'octyle,
le 5,7-dihydro-6H-dibenzo[c,e]azépine-6-carboximidate de 2-propynyle,
le 5,7-dihydro-6H-dibenzo[c,e]azépine-6-carboximidate de cyclohexyle,
le 5,7-dihydro-6H-dibenzo[c,e]azépine-6-carboximidate de cyclopentyle,
le 5,7-dihydro-6H-dibenzo[c,e]azépine-6-carboximidate d'éthyle, 1,5-oxalate, et
le 5,7-dihydro-6H-dibenzo[c,e]azépine-6-carboximidate d'éthyle, diphosphate,
avec des produits auxiliaires de formulation.

9. Procédé de préparation des composés de formule générale

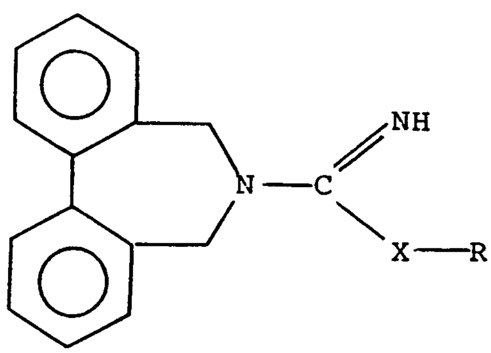

I

dans laquelle
R représente un groupe alkyle en C 1-C 12, alcényle en C 3-C 12, alcynyle en C 3-C 12 ou cycloalkyle en C 3-C 8, et
X représente l'oxygène ou le soufre,
aucune des liaisons multiples éventuelles de R ne devant se trouver en position alpha par rapport à X, et de leurs sels formés par addition avec des acides, caractérisé en ce que l'on fait réagir le 5,7-dihydro-6H-dibenzo[c,e]azépine-6-carbonitrile avec un alcool ou thiol respectivement de formule générale

R-XH III

dans laquelle R et X ont les significations indiquées ci-dessus, ou avec un sel de métal alcalin d'un tel alcool ou thiol, et si on le désire, on convertit un composé ainsi obtenu, répondant à la formule I, en sel formé par addition avec un acide par réaction avec l'acide correspondant.

**10.** Procédé de préparation d'un produit pesticide, caractérisé en ce que l'on mélange au moins un des composés mentionnés dans la revendication 1 avec des produits auxiliaires de formulation.

**11.** Procédé pour combattre les parasites, caractérisé en ce que l'on traite l'objet à protéger ou les parasites eux-mêmes par une quantité efficace d'au moins un composé mentionné dans l'une des revendications 1 à 8 ou d'un produit selon une de ces revendications.

**12.** Utilisation d'un composé mentionné dans l'une des revendications 1 à 8, ou d'un produit selon une de ces revendications, pour la lutte contre les parasites.

**13.** Produit pesticide selon revendication 1, caractérisé en ce qu'il contient une autre substance active consistant en un acaricide prévue pour combattre les acariens non développés, c'est-à-dire les oeufs et les larves.

**14.** Procédé de préparation de composés de formule générale

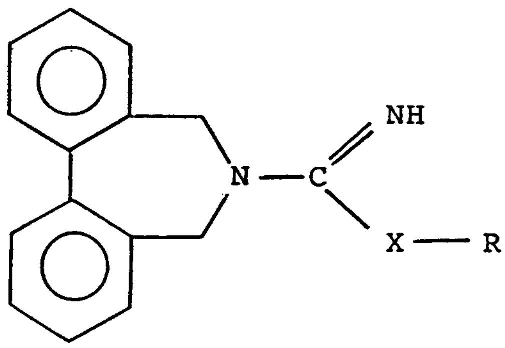

I

dans laquelle
R représente un groupe méthyle ou éthyle, et
X représente l'oxygène ou le soufre,
et de leurs sels formés par addition avec des acides, caractérisé en ce que l'on alkyle une urée ou thiourée respectivement, de formule générale

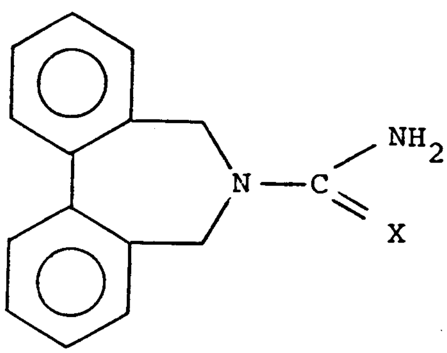

IV

dans laquelle X a les significations indiquées ci-dessus,
et si on le désire, on convertit un composé ainsi obtenu, répondant à la formule I, en sel formé par addition avec un acide par réaction avec l'acide correspondant.